# EUROPEAN PATENT SPECIFICATION

(11) **EP 3 692 345 B1**
(45) Date of publication and mention of the grant of the patent: **14.12.2022**
(21) Application number: 18793472.4
(22) Date of filing: 02.10.2018
(51) Int. Cl.: G01H 1/00, G01N 33/00

(54) **PHYTOSTATIC ANALYSIS DEVICE**
PHYTOSTATISCHE ANALYSEVORRICHTUNG
DISPOSITIF D'ANALYSE PHYTOSTATIQUE

(30) Priority: 03.10.2017 IT 201700110668
(43) Date of publication of application: 12.08.2020
(73) Proprietor: PNAT S.r.l., 50129 Firenze (IT)
(72) Inventor: MANCUSO, Stefano, I-50136 Firenze (IT)
(74) Representative: Lunati & Mazzoni S.r.L.
(86) International application number: PCT/IB2018/057631
(87) International publication number: WO 2019/069219

(56) References cited:
- DE-A1- 19 504 628
- US-A1- 2004 069 064
- US-A1- 2005 234 645
- US-B1- 6 427 535
- TIM VAN EMMERIK ET AL: "Measuring Tree Properties and Responses Using Low-Cost Accelerometers", SENSORS, vol. 17, no. 5, 11 May 2017 (2017-05-11), page 1098, XP055478550, DOI: 10.3390/s17051098
- Swarnalatha Srinivas ET AL: "Eff cient Protection of Palms from RPW Larvae using Wireless Sensor Networks", International Journal of Computer Science Issues (IJCSI), 2 May 2013 (2013-05-02), page 192, XP055167273, Mahebourg Retrieved from the Internet: URL:http://search.proquest.com/docview/147 0969710

## Description

The present invention relates to a phytostatic analysis device of the type specified in the preamble of the first claim.

In particular, the device can analyse the stability of a plant or tree (hereinafter univocally identified with the term "tree") in order to prevent it from falling, preferably by cutting down the plant.

As is known, currently the phytostatic analysis of a tree is normally performed through a visual examination of the tree adapted to examine the characteristics and general condition of the tree and highlight any structural defects therein.

One of the most used visual examinations is the V.T.A. (*Visual Tree Assessment*)*.* This examination is based on the axiom of constant tension, i.e. the fact that biological structures develop so as to ensure even distribution of loads on the surface. Accordingly, V.T.A. teaches that when a tree undergoes rot/breakage, it tends to produce repair material for damaged areas that restore even distribution of loads and produce superficial and therefore visible non-uniformities.

In conclusion, this examination provides a visual investigation aimed at identifying external symptoms of the presence of degenerative processes within the tissues. This investigation is carried out on the foliage, the woody parts (trunk, main branches, and primary branching), and the collar of the tree.

Known phytostatic analysis devices are disclosed in US2005234645, DE19504628 and "Measuring Tree Properties and Response Using Low-Cost Accelerometers" by Tim Van Emerik.

The above-mentioned prior art has a few major drawbacks.

A first drawback is that these examinations require a high level of attention and, therefore, are very long and laborious.

This aspect is emphasized by the fact that the phytostatic analyses must be performed periodically on each tree.

Therefore, another drawback is that a large number of people are required to perform phytostatic analyses of woods with many trees.

A major drawback is that the identification of defects, and therefore the visual investigation requires specialised personnel capable of associating each external symptom with the correct degree of internal degeneration of the tree.

Moreover, despite the use of specialised personnel, visual investigations do not allow accurate phytostatic analyses. Therefore, visual analysis is often accompanied by instrumental investigation.

These instrumental investigations involve the removal of small sections of the trunk and/or other parts of the tree, or alternatively, the use of radiological techniques. These instrumental investigations, although improving the phytostatic analysis, are poorly applied due to the high cost and invasiveness thereof, and because they increase analysis times.

Another drawback is that the investigations possible to date, due to high costs and times, are not applied to all plants of the territory in continuous, and also only allow point coverage.

In this context, the technical task underlying the present invention is to devise a phytostatic analysis device, which is capable of substantially obviating the above-mentioned drawbacks.

Within the scope of said technical task, a major object of the invention is to provide a phytostatic analysis device, which allows a phytostatic analysis to be performed in a precise and fast way.

Another major object of the invention is to obtain a phytostatic analysis device, which is executable in real time and continuously over time without the use of massive human or economic resources.

The technical task and the specified objects are achieved by means of a phytostatic analysis device as claimed in the appended claim 1 and the phytostatic analysis process as claimed in the appended claim 7.

Preferred embodiments are set forth in the dependent claims.

The features and advantages of the invention will be apparent from the detailed description of a preferred embodiment of the invention, with reference to the accompanying drawings, in which:

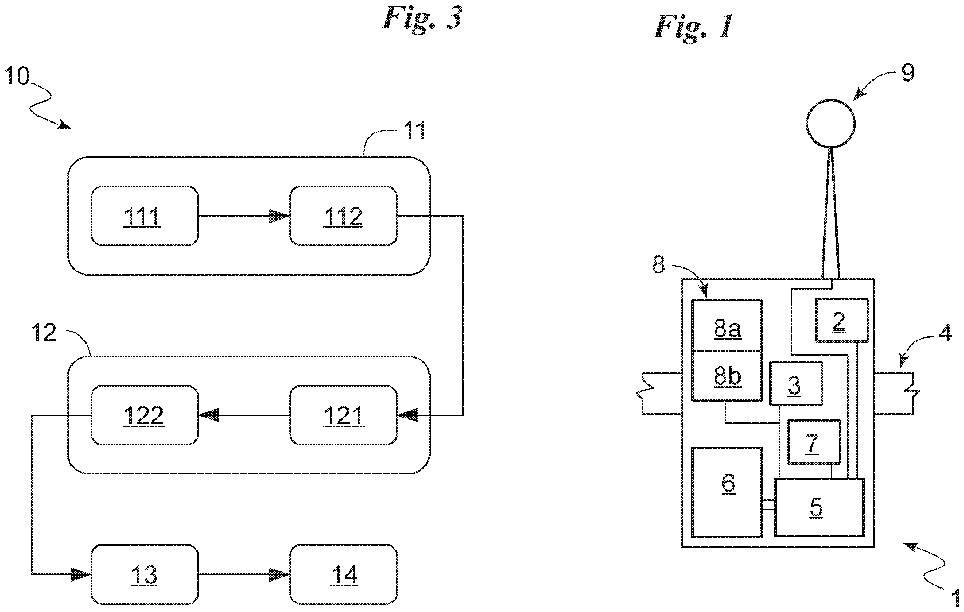

**Fig. 1** shows a phytostatic analysis device according to the invention;
**Fig. 2** shows a phytostatic analysis device in use;
**Fig. 3** schematizes the operation of a phytostatic analysis device according to the invention; and
**Fig. 4** shows a graphical representation of an estimate obtainable by means of the phytostatic analysis device.

In the present document, the measures, values, shapes and geometric references (such as perpendicularity and parallelism), when associated with terms like "about" or other similar terms such as "almost" or "substantially", are to be understood as unless measurement errors or inaccuracies due to production and/or manufacturing defects and, especially, unless a slight difference from the value, measure, shape, or geometric reference with which it is associated. For example, these terms, if associated with a value, preferably indicate a difference not exceeding 10% of the value itself.

Unless otherwise specified, as is apparent from the following discussion, terms such as "treatment", "data processing", "determination", "calculation", or the like, are understood to refer to the action and/or processes of a computer or similar electronic computing device which manipulates and/or transforms data represented as physical, such as electronic sizes of registers of a computer system and/or memories, into other data similarly represented as physical quantities in computer systems, registers or other storage, transmission or information display devices. With reference to the cited Figures, the phytostatic analysis device according to the invention is indicated as a whole by the numeral **1.**

It is adapted to verify, and in particular monitor the stability of a tree **1a,** by determining the phytostatic condition thereof, and preferably report any danger of falling of the tree, thus allowing, for example, the tree to be cut safely.

The phytostatic analysis device 1 may comprise a detection apparatus for detecting the vibrations of the tree 1a, i.e. tree 1a vibrations suitably having a frequency substantially lower than 1 KHz.

Therefore, it should be noted that the phytostatic analysis device 1 uses the detection apparatus to measure the frequencies of the vibrations arising from the earth's crust, which are characterised by a very specific frequency. These frequencies reflect the continuous imperceptible vibration of the earth which, for example, is detected by seismographs as background noise.

It is also noted that the detection apparatus, in addition to measuring the tree 1a vibration frequencies arising from the earth's crust, is also adapted to measure the frequencies caused by any other mechanical agent pressing on the tree 1a, including its root system.

The detection apparatus comprises one or more inertial sensors adapted to detect the vibrations of the tree 1a and suitably having a detection frequency substantially lower than 1 KHz.

In detail, it may comprise at least one accelerometer **2** adapted to be integrally constrained to the tree 1a so as to measure the forces acting thereon.

The accelerometer 2 may have said detection frequency.

It is adapted to detect and/or measure the linear acceleration of the tree 1a. Preferably, the accelerometer 2 is triaxial, thus capable of measuring linear forces/accelerations of the tree 1a along three mutually perpendicular axes.

The detection apparatus may comprise at least one gyroscope 3 adapted to be integrally constrained to the tree 1a so as to measure the moments acting on said tree 1a.

The gyroscope 3 may have said detection frequency.

It is adapted to detect and/or measure the angular acceleration of the tree 1a. Preferably, the gyroscope 3 is triaxial, thus capable of measuring angular moments/accelerations of the tree 1a relative to three mutually perpendicular axes and, in detail, coincident with those of the triaxial accelerometer 2.

Preferably, the detection apparatus comprises only one accelerometer 2 and only one gyroscope 3.

In order to allow the detection apparatus to correctly detect the physical actions acting on the tree 1a, the phytostatic analysis device 1 may comprise anchoring means **4** adapted to integrally constrain the detection apparatus, and precisely the entire device 1 to the tree 1a.

The phytostatic analysis device 1 can comprise a control board **5** controlling the operation of the phytostatic analysis device 1.

The control board 5, as described in greater detail below, is adapted to control the detection apparatus, through its inertial sensors, to measure the vibrations acting on the tree 1a, and optionally to determine the frequency spectrum of said vibrations of said tree 1a as a function of said vibrations.

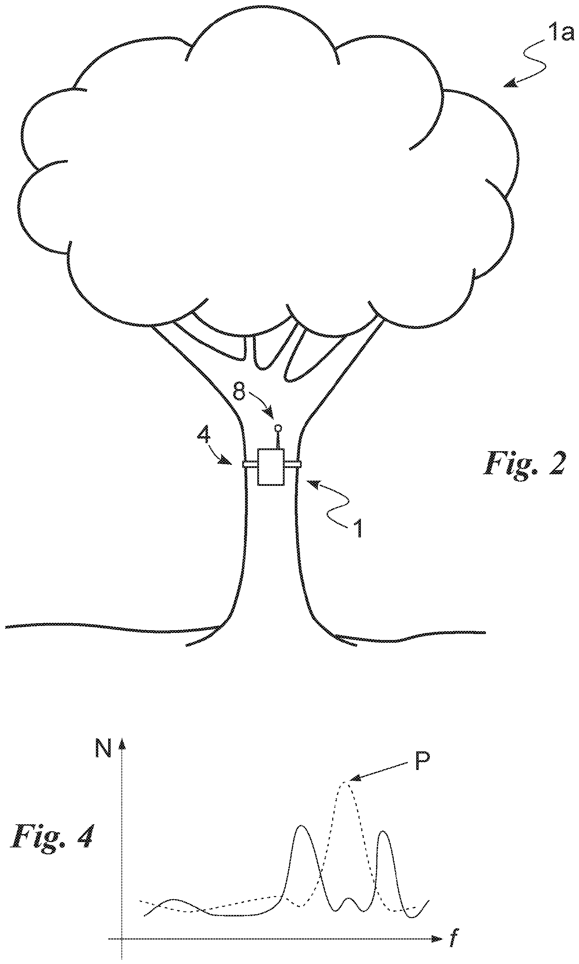

The term spectrum (whether it is a sampling and/or current spectrum) can identify a representation of the vibrations by showing the vibration frequencies vs the number of detections (expressed as absolute or % values) of such frequency, i.e. the number of times said frequency is registered. For example, said spectrum may be a graph as illustrated in Fig. 4, showing on a Cartesian plane the frequency (f) on the abscissa and the number of detections (N) on the ordinate.

In particular, it is adapted to control the detection apparatus (precisely the accelerometer 2 and/or the gyroscope 3) to measure the sampling vibrations acting on the tree 1a, and optionally to determine the sampling spectrum of the tree 1a as a function of said sampling vibrations.

A possible sampling spectrum is shown with a solid line in Fig. 4.

After obtaining the sampling spectrum, the control board 5 is adapted to control the detection apparatus (in particular the accelerometer 2 and/or the gyroscope 3) to measure the current vibrations of the tree 1a (i.e. the vibrations during the phytostatic analysis) and determines, based on the current vibrations, the current spectrum, and lastly the phytostatic condition of the tree 1a by comparing the current spectrum with the sampling spectrum.

A possible current spectrum is shown with a broken line in Fig. 4.

In particular, the control board 5 determines the phytostatic condition of the tree 1a by comparing the sampling spectrum with the current spectrum to search for one or more frequencies at which the detection peaks are recorded (indicated with P in Fig. 4) in only one of the spectra, and to be precise, exclusively within the current spectrum.

Preferably, the phytostatic condition of the tree 1a is determined by comparing the current spectrum with the sampling spectrum within a frequency band substantially lower than 1 KHz.

The phytostatic analysis device 1 may further comprise a power supply system **6** adapted to provide electrical energy to the components of the same phytostatic analysis device 1.

Said power supply system 6 may comprise a battery and/or photovoltaic panels. The phytostatic analysis device 1 may comprise an identifier for the device 1 or the tree 1a.

Said identifier is adapted to allow identification of the device 1 or the tree 1a and may comprise at least one of a geolocator adapted to allow identification of the position of the device 1, and therefore of the tree 1a with which it is associated, and an alphanumeric code of the device 1 or the tree 1a.

Lastly, the phytostatic analysis device 1 may comprise a memory **7** adapted to store data from the same device as described below.

Optionally, the phytostatic analysis device 1 may comprise at least one environmental sensor **8** adapted to measure an atmospheric parameter.

Said at least one environmental sensor 8 may comprise a thermometer **8a** adapted to measure the ambient temperature.

The at least one environmental sensor 8 may comprise a humidity detector **8b** adapted to measure air humidity.

Preferably, the at least one environmental sensor 8 may comprise a thermometer 8a and a humidity detector 8b.

The phytostatic analysis device 1 can be integrated into a phytostatic control system, which is adapted to assess, and in particular to monitor the stability of a plurality of trees 1a.

Such a phytostatic control system comprises one said phytostatic analysis device 1 for each of the trees 1a to be monitored; and a control station comprising a tree database linking an above identifier and one or more sampling and/or current spectra as described below to each tree 1a provided with the phytostatic analysis device 1.

The control station and the one or more phytostatic analysis device(s) 1 is in data connection.

Therefore, each device 1 can comprise connecting means **9** adapted to provide said data connection with the control station.

Obviously, the control station may also include its own connecting means.

The data connection may be wireless and suitably selected from a local network WLAN (*wireless local area network*)*,* a radio mobile cellular network (such as GSM, GPRS, EDGE, UMTS, HSPA, LTE, Bluetooth), or a satellite network.

In some cases, the phytostatic control system may comprise one or more node(s) adapted to interpose, in the data connection, between the control station and the one or more phytostatic analysis device(s) 1. In these cases, the phytostatic analysis devices 1 exchange data with the at least one node (for example via a wireless connection suitably of the Bluetooth type, preferably of the low energy type (Wibree)) and the at least one node with the control station conveniently via a wireless connection, which can be of a type different from or equal to that between the node and the device 1.

The control station is identifiable as a server/processor/computer.

It may be adapted to determine the sampling spectrum as a function of said sampling vibrations measured by a device 1, determine the current spectrum as a function of the current vibrations, and then establish the phytostatic condition of the tree 1a by comparing the current spectrum with the sampling spectrum.

The operation of a phytostatic analysis device, and therefore of a phytostatic control system, described above as regards structure, allows the above mentioned innovative phytostatic analysis method **10** to be defined as at least partially controllable by the control board.

The phytostatic analysis process 10 for phytostatically analysing the tree 1a comprises a sampling step **11** wherein the detection apparatus measures the sampling vibrations on the tree 1a and the sampling spectrum of the tree 1a is determined as a function of said sampling vibrations.

The spectrum of the vibration frequencies of the tree 1a represents the set of frequencies that the tree 1a, when excited, for example, by a vibration of the earth's crust or other external agent on the tree 1a, can take on without this vibration interfering with the stability of the tree 1a causing, for example, its fall or damage. In particular, the sampling step 11 comprises a detection sub-step **111,** wherein the inertial sensors of the detection apparatus, and to be precise the accelerometer 2 and/or the gyroscope 3 measure the sampling vibrations on the tree 1a, and a profiling sub-step **112** for profiling the tree 1a, wherein the sampling spectrum is determined according to said sampling vibrations.

Suitably, the detection sub-step 111 can be performed for an extended period of time (for example for days or weeks) so as to have a plurality of sampling vibrations, which are sufficient to generate the sampling spectrum.

The detection sub-step 111 detects sampling vibrations substantially having a frequency of 1 KHz.

Optionally, in addition to the vibrations in the detection sub-step 111, the environmental sensor 8 can detect at least one selected from the sampling temperature and the air sampling humidity, and preferably both the sampling temperature and the sampling humidity.

The data acquired in this detection sub-step 111 may be stored in the memory of the phytostatic analysis device 1 and/or in the tree database of the control station.

The profiling sub-step 112 determines the sampling spectrum (solid line in Fig. 4) of the tree 1a as a function of the sampling vibrations determined in the earlier detection sub-step 111. The calculation of the sampling spectrum can be carried out by using a Fourier transform, to be precise a fast Fourier transform.

In addition to the sampling vibrations, the sampling spectrum can be determined as a function of the sampling temperature and/or the sampling humidity.

The sampling spectrum can be stored in the tree database of the control station and/or in the phytostatic analysis device 1.

It may take into account sampling vibrations having a frequency substantially lower than 1 KHz.

The profiling sub-step 112 can be performed by the device and\or the control station. It should be noted that the sampling step 11 can be performed periodically (for example once or twice a year) in order to adjust the sampling spectrum to the evolution of the tree 1a.

Once the sampling spectrum has been obtained, the sampling step 11 is complete and the phytostatic analysis method 10 comprises a monitoring step **12,** wherein the current vibrations of the tree 1a, i.e. the vibrations in the tree 1a at the time of monitoring are measured, and the current spectrum is determined on the basis thereof.

In particular, the monitoring step 12 comprises a measuring sub-step **121,** wherein the inertial sensors of the detection apparatus, and to be precise the accelerometer 2 and/or the gyroscope 3 measure the current vibrations acting on the tree 1a, and an analysis sub-step **122,** wherein the current spectrum is determined according to said current vibrations.

The measuring sub-step 121 detects current vibrations having a frequency substantially lower than 1 KHz.

Optionally, in addition to the vibrations in the measuring sub-step 121, the environmental sensor 8 can detect at least one selected from the current temperature and the air current humidity, and preferably both the current temperature and the current humidity.

The analysis sub-step 122 determines the current spectrum (broken line in Fig. 4) of the tree 1a as a function of the current vibrations determined in the earlier measuring sub-step 121. The calculation of the current spectrum can be carried out by using a Fourier transform, to be precise a fast Fourier transform.

In addition to the current vibrations, the sampling spectrum can be determined as a function of the current temperature and/or the current humidity.

The current spectrum can be stored in the tree database of the control station and/or in the phytostatic analysis device 1.

The current spectrum may take into account current vibrations having a frequency substantially lower than 1 KHz.

In order to make the current and sampling spectra comparable to each other, it is possible to adopt solutions such as, for example, by ensuring that the measuring 121 and detection 111 sub-steps have the same duration; and/or by normalizing the number of detections or expressing it as a percentage.

The analysis sub-step 122 can be performed by the device and\or the control station.

Optionally, the monitoring step 12 may comprise an updating sub-step **123,** wherein the sampling spectrum, for example obtained in the sampling step 11, is updated based on the current frequencies suitably measured in the measuring sub-step 121. At the end of the monitoring step 12, the process 10 comprises an assessment step **13,** wherein the phytostatic condition of the tree 1a is determined by comparing the current spectrum with the sampling spectrum.

The assessment step 13 can be performed by the device and\or the control station. The monitoring 12 and assessment 13 steps may be performed at a different, suitably higher rate (for example daily) compared to that of the sampling step 11. In the assessment step 13, the phytostatic condition of the tree 1a can be determined by comparing the current spectrum with the sampling spectrum within a frequency band substantially lower than 1 KHz.

The determination of the phytostatic condition, hence the presence of dangerous alterations in the phytostatic condition of the tree 1a can be carried out by comparing, as in Fig. 4, the sampling and current spectra in search of frequencies (in detail a range of frequencies) in which a peak of detections (i.e. an absolute and/or relative maximum) occurs in only one of the two spectra, to be precise solely in the current spectrum. In other words, the determination of the phytostatic condition is performed by searching for, in the current spectrum, an increase/peak of the number of detections at frequencies where the sampling spectrum does not have a peak of detections (see point P in Fig. 4), and thus records a limited number of detections.

Therefore, a dangerous alteration in the phytostatic condition of the tree 1a occurs if the current spectrum has detection peaks at frequencies different from those where the detection peaks occur in the sampling spectrum. It should be pointed out that increased detections at detection peaks in the current spectrum compared to the sampling spectrum do not define a dangerous alteration in the phytostatic condition.

It can be seen that this difference in the detection peaks, for example, is identified as the occurrence in the current spectrum of a peak not occurring in the sampling spectrum and/or a displacement (referred to as *shifting*) of a peak from one frequency to another, and/or amplitude variations in pre-existing peaks.

Where there is/are one or more dangerous alteration(s) in the phytostatic condition, the phytostatic analysis method 10 may comprise a signalling step **14,** wherein the control station signals the tree 1a at risk of falling.

In detail, the phytostatic analysis device 1 sends the identifier to the control station which signals the tree 1a at risk of falling to the operator.

Alternatively, or additionally, as the assessment step 13 can be executed by the control station, the signalling step may be performed directly by the control station. Finally, it should be noted that in some cases, in the assessment step 13, a comparison can be made between the trees 1a, more precisely between the detections\spectra detected by different devices 1 on different trees 1a. Said assessment of different trees 1a, for example, can compare if any changes in the spectrum are attributable to growth or seasonal changes of the tree 1a, or instead to the occurrence of phytostatic problems in a tree 1a.

Said comparison can be performed between spectra obtained at different times and more appropriately between spectra detected according to a given temporal order (for example, between spectra obtained in subsequent weeks/months).

In addition, the comparison between the spectra can be done by using spectra determined as a function of frequencies detected in the same period of the year (for example, by using sampling and current spectra, both detected in autumn or spring). The monitoring 12 and/or assessment 13 step(s) may be preferably repeated on a monthly and/or yearly basis.

The invention provides significant advantages.

A first advantage is that the phytostatic analysis device 1 allows a very fast and precise assessment of the phytostatic condition of the tree 1a.

In particular, since this assessment is totally automatic (i.e. it does not require the intervention of an operator), the phytostatic analysis device 1 allows the phytostatic condition of a tree 1a to be analysed very quickly.

Moreover, the innovative use of frequencies, and therefore of the frequency spectrum, can determine the presence of internal degenerative processes that alter the phytostatic condition of the tree 1a even without the presence of evident external symptoms or the use of complex and expensive instrumental investigations.

This advantage is due to the fact that the innovative way of using frequencies exploits the manner in which the modification of the structure of the tree 1a caused by internal degenerative processes alters the physical-mechanical characteristics of the tree 1a. Accordingly, the tree 1a changes its response to vibrations (i.e. its vibration mode) thereby modifying what is detected by the detection apparatus and hence the vibration frequencies calculated by the device 1 and the number of times the tree 1a vibrates with these frequencies.

Therefore, the innovative device 1 and the phytostatic analysis process 10 that can be implemented by it exploit the fact that the occurrence of alterations in the internal structure, by altering the physical-mechanical characteristics of the tree 1a - the action acting on the tree 1a being the same - causes the response of the tree 1a with the altered internal structure to be different from that given before the occurrence of this alteration. Therefore, the detection apparatus, and in detail the accelerometer 2 and/or the gyroscope 3 detect a different linear/angular acceleration, thus allowing the calculation of a different vibration frequency and/or a different number of detections of this vibration frequency.

It is also shown that the innovative use of vibrations enables an objective phytostatic analysis device 1 as it is based on the creation\shift of frequency peaks at specific frequencies.

Another advantage is the possibility of remotely controlling and/or viewing a phytostatic analysis of one or more trees.

In fact, the presence of the connecting means of the control station allows an operator to connect remotely (for example, via smartphone) to the control station, therefore to access the tree database or control the execution of one or more analyses.

Another advantage is the memory of the phytostatic analysis device 1 in which to store one or more spectra, and/or the tree database, which make it possible to have a historical list of all the trees 1a subject to analysis and therefore examine the evolution of the tree 1a and analyse how its growth affects the phytostatic condition of the tree 1a.

The invention is susceptible of variations falling within the scope of the inventive concept, as specified in the independent claims, and of the related technical equivalents. In this context, all details are replaceable by equivalent elements and any type of materials, shapes and dimensions may be present.

## Claims

1. A phytostatic control system comprising a plurality of phytostatic analysis devices (1) and a control station in data connection with said phytostatic analysis devices (1); each of said phytostatic analysis devices (1) for the analysis of the phytostatic condition of a tree **characterised in that** it comprises
- a detection apparatus (2, 3) for detecting vibrations of said tree (1a);
- anchoring means (4) for anchoring said detection apparatus (2, 3) to said tree (1a);
- a control board (5) for controlling:
∘ said detection apparatus (2, 3) to measure sampling vibrations of said tree (1a) and said control board (5) determines the sampling spectrum of said tree (1a) as a function of said sampling vibrations; and subsequently
∘ said detection apparatus (2, 3) to measure the current vibrations of said tree (1a) and said control board (5) determines a current spectrum of said tree (1a) as a function of said current vibrations; and then
- said control board (5) determines the phytostatic condition of said tree (1a) by comparing said current spectrum with said sampling spectrum; **and in that** each of said phytostatic analysis devices (1) is adapted to be anchored to different trees, allowing said control station to compare said spectra of said trees in order to detect changes in said spectra attributable to phytostatic problems in at least one of said trees.

2. The phytostatic analysis device (1) according to claim 1, wherein said control board (5) performs said comparison between said sampling spectrum and said current spectrum in order to detect frequencies exclusively having detection peaks within said current spectrum.

3. The phytostatic analysis device (1) according to at least one of the preceding claims, wherein said control board (5) determines said phytostatic condition of said tree (1a) by comparing said current spectrum with said sampling spectrum at frequencies substantially lower than 1 KHz.

4. The phytostatic analysis device (1) according to at least one of the preceding claims, wherein said detection apparatus (2, 3) comprises at least one accelerometer (2) adapted to be integrally constrained to said tree (1a).

5. The phytostatic analysis device (1) according to at least one of the preceding claims, wherein said detection apparatus (2, 3) comprises at least one gyroscope (3) adapted to be integrally constrained to said tree (1a).

6. The phytostatic analysis device (1) according to at least one of the preceding claims, comprising at least one of a humidity detector (8b) adapted to measure the humidity, and a thermometer (8a) adapted to measure the temperature; and wherein said sampling spectrum and said current spectrum are determined as a function of at least one of said temperature and said humidity.

7. A phytostatic analysis process (10) for the analysis of multiple different trees (1a) by a phytostatic control system according to any of the previous claims comprising a plurality of phytostatic analysis devices and a control station in data connection with said phytostatic analysis devices comprising
- a sampling step wherein for each of said trees (1a) the sampling vibrations are measured by the detection apparatus (2, 3) of the respective phytostatic analysis device (1) anchored to the respective tree and the respecive sampling spectrum of the vibration frequencies of the corresponding tree is determined as a function of said sampling vibrations;
- a monitoring step wherein for each of said trees (1a) said respective detection apparatus (2, 3) measures the current vibrations of said tree and a current spectrum of the corresponding tree is determined as a function of said current vibrations; and
- an assessment step wherein for each of said trees (1a) the phytostatic condition of said tree is determined by comparing said current spectrum with said sampling spectrum; and
wherein said assessment step performs a comparison between said spectra of said trees (1a) in order to identify phytostatic problems in at least one said tree (1a).

8. The phytostatic analysis process (10) according to the preceding claim, wherein in said assessment step said phytostatic condition of said tree (1a) is determined by comparing said sampling spectrum and said current spectrum with each other in order to detect frequencies exclusively having detection peaks within said current spectrum.

## Patentansprüche

1. Phytostatische Analysevorrichtung, umfassend eine Vielzahl von phytostatischen Analysevorrichtungen (1) und eine Kontrollstation in Datenverbindung mit den genannten phytostatischen Analysevorrichtungen (1); wobei jede der genannten phytostatischen Analysevorrichtungen (1) zur Analyse des phytostatischen Zustands eines Baumes **dadurch gekennzeichnet ist,** Folgendes zu umfassen
- ein Messgerät (2, 3) zum Messen von Schwingungen des genannten Baumes (1a);
- Verankerungsmittel (4) zum Befestigen des genannten Messgeräts (2, 3) an dem genannten Baum (1a);
- eine Steuerkarte (5), die geeignet ist, Folgendes zu befehlen:
∘ dem genannten Messgerät (2, 3), auf den Baum (1a) wirkende Stichprobenschwingungen zu messen, wobei die genannte Steuerkarte (5) das Stichprobenspektrum des genannten Baumes (1a) abhängig von den genannten Stichprobenschwingungen bestimmt; und anschließend
∘ dem genannten Messgerät (2, 3), die auf den Baum (1a) wirkenden aktuellen Schwingungen zu messen, wobei die genannte Steuerkarte (5) ein aktuelles Spektrum des genannten Baumes (1a) abhängig von den genannten aktuellen Schwingungen bestimmt; und
- die genannte Steuerkarte (5) den phytostatischen Zustand des genannten Baumes (1a) durch den Vergleich des genannten aktuellen Spektrums mit dem Stichprobenspektrum bestimmt;
**und dadurch, dass**
- jede der genannten phytostatischen Analysevorrichtungen (1) geeignet ist, an verschiedenen Bäumen befestigt zu werden und es der genannten Kontrollstation zu gestatten, die Spektren der genannten Bäume zu vergleichen, um so phytostatischen Problemen an mindestens einem der genannten Bäume zuzuschreibende Veränderungen der genannten Spektren zu messen.

2. Phytostatische Analysevorrichtung (1) nach Anspruch 1, bei der die genannte Steuerkarte (5) den genannten Vergleich zwischen dem genannten Stichprobenspektrum und dem genannten aktuellen Spektrum ausführt, um Frequenzen festzustellen, die Spitzen von Messungen ausschließlich in dem genannten aktuellen Spektrum aufweisen.

3. Phytostatische Analysevorrichtung (1) nach mindestens einem der vorangegangenen Ansprüche, bei der die genannte Steuerkarte (5) den genannten phytostatischen Zustand des genannten Baumes (1a) bestimmt, indem das genannte aktuelle Spektrum mit dem genannten Stichprobenspektrum bei den im Wesentlichen unter 1 KHz liegenden Frequenzen vergleicht.

4. Phytostatische Analysevorrichtung (1) nach mindestens einem der vorangegangenen Ansprüche, bei der das genannte Messgerät (2, 3) mindestens einen Beschleunigungsmesser (2) umfasst, der geeignet ist, fest an dem genannten Baum (1a) befestigt zu werden.

5. Phytostatische Analysevorrichtung (1) nach mindestens einem der vorangegangenen Ansprüche, bei der das genannte Messgerät (2, 3) mindestens ein Gyroskop (3) umfasst, das geeignet ist, fest an dem genannten Baum (1a) befestigt zu werden.

6. Phytostatische Analysevorrichtung (1) nach mindestens einem der vorangegangenen Ansprüche, umfassend mindestens entweder einen Feuchtigkeitsmesser (8b), der geeignet ist, die Feuchtigkeit zu messen, oder ein Thermometer (8a), das geeignet ist, die Temperatur zu messen; und bei der das genannte Stichprobenspektrum und das genannte aktuelle Spektrum abhängig von mindestens entweder der genannten Temperatur oder der genannten Feuchtigkeit bestimmt werden.

7. Phytostatisches Analyseverfahren (10) zur Analyse einer Vielzahl verschiedener Bäume (1a) mit einer phytostatischen Kontrollanlage nach mindestens einem der vorangegangenen Ansprüche, umfassend eine Vielzahl von phytostatischen Analysevorrichtungen (1) und eine Kontrollstation in Datenverbindung mit den genannten phytostatischen Analysevorrichtungen (1); umfassend
- einen Schritt der Stichprobennahme, bei dem für jeden der genannten Bäume (1a) die Stichprobenschwingungen von dem genannten Messgerät (2, 3) der an dem jeweiligen Baum befestigten jeweiligen phytostatischen Analysevorrichtung (1) gemessen werden und das Stichprobenspektrum der Schwingungsfrequenzen des jeweiligen Baumes (1a) abhängig von den genannten Stichprobenschwingungen bestimmt wird;
- einen Schritt der Überwachung, bei dem für jeden der genannten Bäume (1a) das genannte jeweilige Messgerät (2, 3) die aktuellen auf den genannten Baum wirkenden Schwingungen misst und ein aktuelles Spektrum des genannten jeweiligen Baumes (1a) abhängig von den genannten aktuellen Schwingungen bestimmt wird; und
- einen Schritt der Kontrolle, bei dem für jeden der genannten Bäume (1a) der phytostatische Zustand des genannten Baumes (1a) bestimmt wird, indem das genannte aktuelle Spektrum mit dem genannten Stichprobenspektrum verglichen wird; wobei bei dem genannten Kontrollschritt eine Gegenüberstellung der Spektren der genannten Bäume erfolgt, um so phytostatische Probleme an mindestens einem der genannten Bäume festzustellen.

8. Phytostatisches Analyseverfahren (10) nach dem vorangegangenen Anspruch, bei dem bei dem genannten Kontrollschritt der genannte phytostatische Zustand des genannten Baumes (1a) bestimmt wird, indem das genannte Stichprobenspektrum und das genannte aktuelle Spektrum miteinander verglichen werden, um Frequenzen festzustellen, die Spitzen von Messungen ausschließlich in dem genannten aktuellen Spektrum aufweisen.

## Revendications

1. Installation de contrôle phytostatique comprenant une pluralité de dispositifs d'analyse phytostatique (1) et une station de contrôle échangeant des données avec lesdits dispositifs d'analyse phytostatique (1) ; chacun desdits dispositifs d'analyse phytostatique (1) pour l'analyse des conditions phytostatiques d'un arbre étant **caractérisé en ce qu'il** comprend
- un appareil de détection (2, 3) de vibrations dudit arbre (1a) ;
- des moyens d'ancrage (4) dudit appareil de détection (2, 3) audit arbre (1a) ;
- une carte de commande (5) apte à commander :
∘ audit appareil de détection (2, 3) de mesurer des vibrations d'échantillonnage agissant sur ledit arbre (1a) et ladite carte de commande (5) détermine le spectre d'échantillonnage dudit arbre (1a) en fonction desdites vibrations d'échantillonnage ; et postérieurement
∘ audit appareil de détection (2, 3) de mesurer les vibrations courantes agissant sur ledit arbre (1a) et ladite carte de commande (5) détermine un spectre courant dudit arbre (1a) en fonction desdites vibrations courantes ; et
- ladite carte de commande (5) détermine l'aperçu phytostatique dudit arbre (1a) en comparant ledit spectre courant avec ledit spectre d'échantillonnage ;
**et en ce que**
- chacun desdits dispositifs d'analyse phytostatique (1) est apte à être lié à des arbres différents en permettant à ladite station de contrôle de comparer les spectres desdits arbres de façon à détecter des changements dans lesdits spectres qui peuvent être associés à des problèmes d'un point de vue phytostatique dans au moins un desdits arbres.

2. Dispositif d'analyse phytostatique (1) selon la revendication 1, dans lequel ladite carte de commande (5) effectue ladite comparaison entre ledit spectre d'échantillonnage et ledit spectre courant afin de détecter des fréquences ayant des pics de relèvement exclusivement dans ledit spectre courant.

3. Dispositif d'analyse phytostatique (1) selon au moins une revendication précédente, dans lequel ladite carte de commande (5) détermine ledit aperçu phytostatique dudit arbre (1a) en comparant ledit spectre courant avec ledit spectre d'échantillonnage au niveau des fréquences sensiblement inférieures à 1 KHz.

4. Dispositif d'analyse phytostatique (1) selon au moins une revendication précédente, dans lequel ledit appareil de détection (2, 3) comprend au moins un accéléromètre (2) apte à être lié solidairement audit arbre (1a).

5. Dispositif d'analyse phytostatique (1) selon au moins une revendication précédente, dans lequel ledit appareil de détection (2, 3) au moins un gyroscope (3) apte à être lié solidairement audit arbre (1a).

6. Dispositif d'analyse phytostatique (1) selon au moins une revendication précédente, comprenant au moins l'un d'un détecteur d'humidité (8b) apte à mesurer l'humidité et d'un thermomètre (8a) apte à mesurer la température ; et dans lequel ledit spectre d'échantillonnage et ledit spectre courant sont déterminés en fonction d'au moins un entre ladite température et ladite humidité.

7. Procédé d'analyse phytostatique (10) pour l'analyse d'une pluralité d'arbres différents (1a) avec une installation de contrôle phytostatique selon au moins une revendication précédente comprenant une pluralité de dispositifs d'analyse phytostatique (1) et une station de contrôle échangeant des données avec lesdits dispositifs d'analyse phytostatique (1) ; comprenant
- une phase d'échantillonnage dans laquelle pour chacun desdits arbres (1a) les vibrations d'échantillonnage sont mesurées par ledit appareil de détection (2, 3) du dispositif d'analyse phytostatique (1) respectif lié à l'arbre associé et le spectre d'échantillonnage des fréquences de vibration de l'arbre (1a) associé est déterminé en fonction desdites vibrations d'échantillonnage ;
- une phase de suivi dans laquelle pour chacun desdits arbres (1a) ledit appareil de détection (2, 3) respectif mesure les vibrations courantes agissant sur ledit arbre et un spectre courant dudit arbre (1a) correspondant est déterminé en fonction desdites vibrations courantes ; et
- une phase de contrôle dans laquelle pour chacun desdits arbres (1a) l'aperçu phytostatique dudit arbre (1a) est déterminé en comparant ledit spectre courant avec ledit spectre d'échantillonnage ; et dans laquelle ladite phase de contrôle effectue une comparaison entre les spectres desdits arbres de façon à détecter des problèmes d'un point de vue phytostatique dans au moins un desdits arbres.

8. Procédé d'analyse phytostatique (10) selon la revendication précédente, dans lequel dans ladite phase de contrôle ledit aperçu phytostatique dudit arbre (1a) est déterminé en comparant ledit spectre d'échantillonnage avec ledit spectre courant afin de détecter des fréquences ayant des pics de relèvement exclusivement dans ledit spectre courant.
